(19) Europäisches Patentamt / European Patent Office / Office européen des brevets

(11) **EP 2 687 233 A1**

(12) **EUROPEAN PATENT APPLICATION**
published in accordance with Art. 153(4) EPC

(43) Date of publication:
    **22.01.2014  Bulletin 2014/04**

(21) Application number: **12757846.6**

(22) Date of filing: **15.03.2012**

(51) Int Cl.:
    **A61K 47/02** (2006.01)   **A61K 9/20** (2006.01)
    **A61K 47/26** (2006.01)   **A61K 47/32** (2006.01)
    **A61K 47/36** (2006.01)   **A61K 47/38** (2006.01)

(86) International application number:
    **PCT/JP2012/057414**

(87) International publication number:
    **WO 2012/124827 (20.09.2012 Gazette 2012/38)**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**

(30) Priority: **17.03.2011  JP 2011058993
                05.04.2011  JP 2011083340**

(71) Applicant: **Kyowa Chemical Industry Co., Ltd
    Takamatsu-shi, Kagawa 761-0113 (JP)**

(72) Inventor: **TACHIFUJI Tomoko
    Sakaide-shi, Kagawa 762-0012 (JP)**

(74) Representative: **Raynor, Stuart Andrew
    J A Kemp
    14 South Square
    Gray's Inn
    London WC1R 5JJ (GB)**

(54) **BINDER FOR TABLET FORMING**

(57)    The present invention relates to a novel binder having high bondability and fluidity. The binder of the present invention has a BET specific surface area of 80 to 400 m$^2$/g and is composed of carbonate-containing magnesium hydroxide particles represented by the following Formula (1):

$$Mg(OH)_{2-x}(CO_3)_{0.5x} \cdot mH_2O \qquad (1)$$

wherein, x and m satisfy the following requirements:

$$0.02 \le x \le 0.7,$$

and

$$0 \le m \le 1.$$

Even if the binder of the present invention is prescribed together with a disintegrant or a water-soluble excipient, the binder can improve the tablet hardness without elongating the disintegration time of the tablet and can be applied to direct compression.

EP 2 687 233 A1

**Description**

Technical Field

The present invention relates to a binder used for providing bondability in production of a solid preparation such as a drug, a foodstuff, or an agricultural chemical and relates to a compression molded product produced using the binder. The invention also relates to a disintegrating compression molded product containing the binder together with a disintegrant or a water-soluble excipient.

Background Art

Many of solid preparations such as drugs and foodstuffs are molded using binders having a function of bonding powder particles to one another for increasing the yields in production processes and for increasing the mechanical strengths of molded products. Examples of the binder contained in these solid preparations include starch, gelatin, gum arabic, xanthan gum, dextrin, dextran, pullulan, polyvinyl alcohol, polyvinyl pyrrolidone, hydroxypropyl cellulose, hydroxypropyl methyl cellulose, methyl cellulose, sodium carboxymethylcellulose, and crystalline cellulose. Thus, a variety of organic binders are known, but some of them are unsuitable for direct compression, some of them make the surface of a tablet sticky when the tablet is taken in with a small amount of water and may make the tablet adhere to the esophageal mucosa, some of them may cause allergy symptoms, or some of them are expensive.

In contrast, the types of inorganic binders (excipients) are less compared with the organic binders, and examples thereof include calcium hydrogen phosphate, magnesium aluminometasilicate, and hydrotalcite.

Patent Literature 1 discloses flake-like calcium hydrogen phosphate having excellent bondability prepared by reacting phosphoric acid and alkaline calcium in the presence of a polyvalent organic acid to produce columnar calcium hydrogen phosphate and subjecting the columnar calcium hydrogen phosphate to hydrothermal treatment at 60°C or more. It is described that the flake-like calcium hydrogen phosphate has a BET specific surface area of 20 to 60 $m^2/g$ and thereby is an excipient having excellent bondability, whereas commercially available conventional calcium hydrogen phosphate has a BET specific surface area of 1 $m^2/g$ or less.

Magnesium aluminometasilicate and hydrotalcite are commercially available as excipients having antacid activity. As for magnesium aluminometasilicate and hydrotalcite having high BET specific surface areas, both BET specific surface areas are about 150 $m^2/g$, and highly excellent moldability is provided. However, they are compounds containing aluminum, which may not be liked by consumers.

In magnesium compounds, magnesium silicate has a high BET specific surface area, which is up to 600 $m^2/g$ or more. Though magnesium silicate has a high specific surface area, the strength of bonding particles is low, and therefore magnesium silicate is not suitable as an excipient or a binder.

Patent Literature 2 describes that a carbonate-containing magnesium hydroxide having a high BET specific surface area can be prepared by reacting a magnesium salt solution and an alkaline material in the presence of $CO_3$ ions. The carbonate-containing magnesium hydroxide has a BET specific surface area of 80 $m^2/g$ or more, and a BET specific surface area can also be increased to 200 $m^2/g$ or more (Patent Literature 2).

In medicinal use, basic substances such as magnesium hydroxide are mainly used in antacids, but also can be used as additives for stabilizing agents unstable to acids. Patent Literature 3 discloses an orally rapidly disintegrable tablet that contains water-soluble sugar alcohol and fine particles composed of an acid-unstable benzimidazole-based compound and an inorganic salt of magnesium or calcium and coated with an enteric coating layer.

Patent Literatures 4 and 5 disclose pharmaceutical preparations having excellent storage stability composed of a mixture of a benzimidazole-based compound and magnesium oxide.

Similarly, in Patent Literature 6, agents unstable to acids are stabilized with magnesium hydroxide.

Patent Literature 7 discloses a rapidly absorbable oral administration preparation in which the absorbability of diphenhydramine or its acid addition salt is safely improved by mixing the diphenhydramine or its acid addition salt with an antacid. Such an inorganic basic substance has an effect that cannot be achieved by neutral substances such as crystalline cellulose, but there is no magnesium compound that can improve the bondability of a molded product with a small amount thereof.

Citation List

Patent Literature

Patent Literature 1: Japanese Patent Laid-Open No. Hei 7-118005

Patent Literature 2: International Publication No. WO2008/123566
Patent Literature 3: Japanese Patent No. 3746167
Patent Literature 4: Japanese Patent Laid-Open No. 2009-209048
Patent Literature 5: Japanese Patent Laid-Open No. 2010-47553
Patent Literature 6: Japanese Patent Laid-Open No. 2008-255088
Patent Literature 7: Japanese Patent Laid-Open No. 2008-174500

Disclosure of the Invention

It is an object of the present invention to provide a binder that is effective as a binder being used in molding of a solid preparation such as a drug or a foodstuff and has excellent fluidity and does not delay the disintegration of the solid preparation.

In order to achieve the object, the present inventors focused on carbonate-containing magnesium hydroxide particles having a high BET specific surface area and investigated usefulness of the particles as a binder in production of a solid preparation. As a result, it was found that though carbonate-containing magnesium hydroxide particles having a high BET specific surface area are a magnesium compound, the particles have high bondability equivalent to or higher than that of crystalline cellulose. In addition, it was found that a binder consisting of the carbonate-containing magnesium hydroxide particles does not delay the disintegration of a solid preparation when the binder is used with a disintegrant. Thus, the present invention has been accomplished.

The present invention relates to a binder having a BET specific surface area of 80 to 400 $m^2$/g and comprising carbonate-containing magnesium hydroxide particles represented by the following Formula (1):

$$Mg(OH)_{2-x}(CO_3)_{0.5x} \cdot mH_2O \qquad (1)$$

wherein, x and m satisfy the following requirements:

$$0.02 \leq x \leq 0.7,$$

and

$$0 \leq m \leq 1.$$

Description of Embodiments

<Binder>

The binder of the present invention is composed of carbonate-containing magnesium hydroxide particles represented by the following Formula (1):

$$Mg(OH)_{2-x}(CO_3)_{0.5x} \cdot mH_2O \qquad (1)$$

wherein, x and m satisfy the following requirements:

$$0.02 \leq x \leq 0.7,$$

and

$$0 \leq m \leq 1.$$

In the formula, x satisfies a requirement of $0.02 \leq x \leq 0.7$, preferably $0.04 \leq x \leq 0.6$, and more preferably $0.06 \leq x \leq 0.3$. In the formula, m satisfies a requirement of $0 \leq m \leq 1$ and preferably $0 \leq m \leq 0.5$.

The binder of the present invention has a BET specific surface area of 80 to 400 $m^2$/g. The lower limit of the BET specific surface area of the binder of the present invention is 80 m$^2$/g, preferably 100 m$^2$/g, and more preferably 120 m$^2$/g.

The upper limit of the BET specific surface area is 400 m$^2$/g, preferably 350 m$^2$/g, and more preferably 300 m$^2$/g. The binder of the present invention preferably has a BET specific surface area within a range of 80 to 350 m$^2$/g.

Though the particles of the binder of the present invention contain about 0.75 to 23 wt% of $CO_3$ ions in $CO_2$ equivalent, the x-ray diffraction image and the differential thermal analysis (DTA) of the binder show characteristics specific to magnesium hydroxide, and the BET specific surface area is 80 to 400 m$^2$/g. A higher content of $CO_3$ ions further inhibits the crystal growth of the magnesium hydroxide particles and provides a higher BET specific surface area to the resulting magnesium hydroxide particles. However, if the $CO_2$ content exceeds 23 wt%, the generation of magnesium carbonate is confirmed by the x-ray diffraction image and the differential thermal analysis (DTA), and, undesirably, $CO_3$ ions work so as to accelerate the crystal growth of magnesium carbonate to reduce the BET specific surface area.

The binder of the present invention has an average particle diameter of 5 to 1000 $\mu$m. In a case of molding by direct compression, from the viewpoints of fluidity and uniform distribution, the average particle diameter is preferably 20 to 500 $\mu$m and more preferably 50 to 200 $\mu$m.

<Method of producing magnesium hydroxide particles>

The magnesium hydroxide particles constituting the binder of the present invention can be produced by bringing Mg ions and OH ions into contact with each other in water in the presence of $CO_3$ ions.

Mg ions are preferably used in a form of an aqueous solution of a magnesium salt. Examples of the magnesium salt include magnesium chloride (including bittern from which Ca is removed), magnesium sulfate, magnesium nitrate, and magnesium acetate.

Since the reaction system contains $CO_3$ ions in order to inhibit crystal growth of magnesium hydroxide particles, the aqueous solution of a magnesium salt preferably contains Ca ions as less as possible. If a reaction system contains Ca ions, the Ca ions react with $CO_3$ ions added for inhibiting crystal growth of magnesium hydroxide particles to generate $CaCO_3$. In addition, a divalent anion, $SO_4$ ion, has a function of inhibiting crystal growth of magnesium hydroxide particles like $CO_3$ ions do. Accordingly, when the aqueous solution of a magnesium salt is an aqueous magnesium sulfate solution, though the reaction of only sodium hydroxide and an aqueous magnesium sulfate solution can provide magnesium hydroxide particles having a specific surface area enlarged to some extent, and the presence of $CO_3$ ions can further remarkably increase the specific surface area.

The OH ion is preferably used in a form of an aqueous solution of, for example, an alkali metal hydroxide or ammonium hydroxide. The alkali metal hydroxide is preferably sodium hydroxide.

The $CO_3$ ions can be supplied from an aqueous solution of a carbonate such as alkali metal carbonate or ammonium carbonate or from a form of $CO_2$ gas, and an aqueous solution of a carbonate is preferred for controlling the abundance ratio of OH ions to $CO_3$ ions.

The magnesium hydroxide particles are preferably produced by bringing an aqueous magnesium salt solution and an aqueous solution of an alkali metal hydroxide into contact with each other in the presence of an alkali metal carbonate.

In the production of the magnesium hydroxide particles, since the abundance ratio of OH ions to $CO_3$ ions determines the chemical composition of the binder, the abundance ratio of OH ions to $CO_3$ ions at the reaction is essential. If the amount of $CO_3$ ions is high, as is obvious, magnesium carbonate is generated. The presence of the magnesium carbonate is confirmed by the x-ray diffraction image and the differential thermal analysis (DTA) and leads to a reduction in the specific surface area of the generated magnesium hydroxide particles. The results of research by the present inventors demonstrate that within a molar ratio range of 2(OH):$CO_3$ = 99:1 to 65:35, the magnesium hydroxide particles have a high specific surface area and show characteristics of magnesium hydroxide particles in the x-ray diffraction image and the differential thermal analysis (DTA). The molar ratio is preferably 2(OH):$CO_3$ = 98:2 to 70:30 and more preferably 2(OH):$CO_3$ = 97:3 to 75:25.

$CO_3$ ions are incorporated into magnesium hydroxide particles together with OH ions when the particles are generated and thereby inhibit the crystal growth of the magnesium hydroxide particles. Therefore, in the case of adding $CO_3$ ions to magnesium hydroxide particles after generation of the particles, magnesium hydroxide particles having a high BET specific surface area as in the present invention cannot be obtained. Accordingly, it is important to stably supply OH ions and $CO_3$ ions at a constant ratio. In order to achieve such supply, it is preferable to prepare a mixed solution of OH ions and $CO_3$ ions and to use the solution in the reaction. Examples of the mixed solution of OH ions and $CO_3$ ions include a mixed aqueous solution of sodium hydroxide and sodium carbonate and a mixed aqueous solution of ammonia and ammonium carbonate. In the light of the yield of magnesium hydroxide particles, a mixed aqueous solution of sodium hydroxide and sodium carbonate is preferred.

The magnesium hydroxide particles of the present invention can also be prepared by continuously pouring OH ions and $CO_2$ gas simultaneously to an aqueous magnesium salt solution with stirring. In such a case, it is important to maintain the abundance ratio of OH ions to $CO_3$ ions constant in the reaction system by controlling the $CO_2$ gas concentration and the flow rate.

The reaction temperature is preferably 0 to 100°C and more preferably 10 to 80°C. The reaction time is preferably 120 min or less and more preferably 60 min or less. The binder can be produced through, for example, continuous stirred tank reaction by continuously supplying an aqueous solution of a magnesium salt (Mg ion) and a mixed aqueous solution of sodium hydroxide (OH ion) and sodium carbonate ($CO_3$ ion) to a reaction tank and continuously extracting the product from the reaction tank. The retention time in this reaction is preferably 120 min or less and more preferably 60 min or less.

The binder can also be produced through batch reaction by adding a mixed aqueous solution of sodium hydroxide (OH ion) and sodium carbonate ($CO_3$ ion) to an aqueous solution of a magnesium salt (Mg ion) in a reaction tank.

In a case of using magnesium chloride (including bittern from which Ca is removed) or magnesium sulfate as the magnesium salt solution in raw materials, the magnesium hydroxide particles prepared by the reaction contain Cl ions and $SO_4$ ions as impurities in some cases. The content of Cl ions is preferably 0.5 wt% or less and more preferably 0.3 wt% or less. The content of $SO_4$ ions is preferably 2 wt% or less and more preferably 1.5 wt% or less.

The slurry product prepared by the reaction is preferably subjected to filtration, washed with water or a dilute alkaline aqueous solution, and then dried. The drying can be performed by shelf-type hot-air drying, spray drying or the like. The drying in this case is preferably performed at 80 to 250°C for removing water. Alternatively, vacuum drying can be performed by replacing water by an organic solvent without applying heat. The shelf-type hot-air dried product or the vacuum dried product is in a massive form and is preferably pulverized into powder depending on the intended use.

<Granulated binder>

The binder can be formed into a granulated binder by granulation. The granulated binder preferably has an average secondary particle diameter of 20 to 1000 $\mu$m and more preferably 20 to 500 $\mu$m.

The granulation can be achieved by spray drying of a slurry of the binder.

The slurry concentration subjected to the spray drying is not specifically limited, but a too low concentration decreases the production capacity, whereas a too high concentration excessively increases the viscosity of the slurry to inhibit solution sending. Accordingly, the solid concentration is preferably 10 to 1000 g/L and more preferably 100 to 500 g/L. The binder of the present invention has excellent granulating ability and can thereby be granulated into highly spherical particles by spray drying even in the absence of another binder. Since the average secondary particle diameter of the spherical particles increases with the slurry concentration, the particle diameter can be controlled to some extent by the slurry concentration.

The spray drying can be performed by a known method. Spray drying using a nozzle provides large particles, whereas spray drying using an atomizer provides small particles.

The granulation can also be performed by subjecting the binder to dry granulation using a roller compactor or wet granulation using an extrusion granulator.

<Compression molded product (solid preparation)>

The present invention encompasses a compression molded product containing at least one binder described above.

The compression molded product containing the binder of the present invention has excellent strength and is therefore not easily disintegrated if no disintegrant is present. In order to gradually release a water-soluble drug efficacy ingredient in the use of, for example, an agricultural chemical or fertilizer, the shape of a molded product can be maintained over a long time by compression molding a composition not containing any disintegrant.

In contrast, in the use of, for example, a drug or a foodstuff requiring rapid disintegration in the stomach and intestines or in water, it is preferable to contain a disintegrant. Examples of the disintegrant include starch, croscarmellose sodium, crospovidone, carmellose calcium, carmellose, low-substituted hydroxypropyl cellulose, and carboxymethyl starch sodium. The content of the disintegrant is preferably 5 to 150 parts by weight, more preferably 10 to 100 parts by weight, based on 100 parts by weight of the binder.

In order to provide a disintegrating property without using any disintegrant, it is preferable to contain a water-soluble excipient. Examples of the water-soluble excipient include sugar, starch, sugar alcohol, and water-soluble salts. The content of the water-soluble excipient is preferably 10 to 1000 parts by weight, more preferably 50 to 800 parts by weight, based on 100 parts by weight of the binder.

The compression molded product may be produced by any method, and direct compression is preferred from the viewpoint of improving operation efficiency. The compression molded product preferably has a tablet strength of 20 N or more and more preferably 30 N or more. The compression molded product preferably has a friability of 0.5% or less.

The present invention encompasses a compression molded product (solid preparation) containing the binder and a drug efficacy ingredient. Examples of the drug efficacy ingredient include those unstable to acids. Examples of the acid-unstable drug efficacy ingredient include benzimidazole-based compounds.

<Method of using as binder>

The present invention encompasses a method of using carbonate-containing magnesium hydroxide particles having a BET specific surface area of 80 to 400 $m^2/g$ and represented by the following Formula (1) as a binder for a compression molded product such as a solid preparation:

$$Mg(OH)_{2-x}(CO_3)_{0.5x} \cdot mH_2O \qquad (1)$$

wherein, x and m satisfy the following requirements:

$$0.02 \leq x \leq 0.7,$$

and

$$0 \leq m \leq 1.$$

This method includes the steps of

(i) preparing carbonate-containing magnesium hydroxide particles represented by Formula (1);
(ii) mixing the particles with at least one ingredient selected from the group consisting of disintegrants, water-soluble excipients, and drug efficacy ingredients; and
(iii) tableting the resulting mixture.

The carbonate-containing magnesium hydroxide particles preferably have an average secondary particle diameter of 1 to 1000 $\mu$m.

Grains prepared by granulation of the carbonate-containing magnesium hydroxide particles can also be used. The granulation is preferably performed by spray drying of a slurry or dry or wet granulation. The granulated grains preferably have an average secondary particle diameter of 20 to 1000 $\mu$m.

The compression molded product preferably contains a disintegrant. The disintegrant is preferably at least one selected from the group consisting of starch, croscarmellose sodium, crospovidone, carmellose calcium, carmellose, low-substituted hydroxypropyl cellulose, and carboxymethyl starch sodium.

The compression molded product preferably contains a water-soluble excipient. The water-soluble excipient is preferably sugar, starch, sugar alcohol, or a water-soluble salt.

The compression molded product is preferably formed by direct compression and has a tablet strength of 20 N or more. The compression molded product is preferably formed by direct compression and has a tablet strength of 30 N or more. The compression molded product is preferably formed by direct compression and has a friability of 0.5% or less.

The compression molded product preferably contains a drug efficacy ingredient. The drug efficacy ingredient may be unstable to acids. The content of the carbonate-containing magnesium hydroxide particles represented by Formula (1) in the compression molded product is preferably 5 to 25% by weight and more preferably 10 to 20% by weight.

Examples

The gist of the present invention will now be described in more detail by Examples and Comparative Examples, but the present invention is not limited to these Examples. The composition and the solid preparation prepared in each example were subjected to the following performance evaluation.

BET specific surface area:

The BET specific surface area of each sample was measured by a BET method with NOVA2000 manufactured by QUANTACHROME Corporation.

Average secondary particle diameter:

The average secondary particle diameter of each sample was measured by a laser diffraction scattering method with MT3300EX II manufactured by MICROTRAC, Inc. Angle of repose:

The angle of repose of each sample was measured with an apparatus of measuring angle of repose, model AOR-57, (manufactured by Tsutsui Scientific Instruments Co., Ltd.).

Tablet hardness:

The tablet hardness of each sample was measured with a hardness meter, model 8M (ver. 4.11), (manufactured by Schleuniger Pharmatron Inc.). The measurement was repeated ten times, and the average thereof was determined.

Disintegration test:

The disintegration of each sample was measured in accordance with the "Disintegration Test" of the Japanese Pharmacopoeia Fifteenth Edition. The measurement was repeated six times, and the average thereof was determined.

Friability test:

The friability of each sample was measured in accordance with the "Tablet Friability Test" of the Japanese Pharmacopoeia Fifteenth Edition with a tablet friability tester (manufactured by Kayagaki Irika Kogyo K.K.).

Examples

Example 1

A slurry prepared by continuous pouring reaction of an aqueous magnesium sulfate solution and an alkaline mixture solution ($NaOH:Na_2CO_3$ = 18:1) was washed with water, dried, and pulverized to yield a binder composed of particles consisting of carbonate-containing magnesium hydroxide represented by $Mg(OH)_{1\cdot8}(CO_3)_{0.1}\cdot0.13H_2O$. The resulting binder had a BET specific surface area of 251 $m^2/g$, an average secondary particle diameter of 12.8 $\mu$m, and an angle of repose of 44°.

Example 2

A slurry prepared by continuous pouring reaction of an aqueous magnesium sulfate solution and an alkaline mixture solution ($NaOH:Na_2CO_3$ = 18:1) was washed with water, emulsified, and then spray-dried to yield a spherical binder composed of particles consisting of carbonate-containing magnesium hydroxide represented by $Mg(OH)_{1\cdot8}$ $(CO_3)_{0\cdot1}\cdot0.18H_2O$. The resulting binder had a BET specific surface area of 237 $m^2/g$, an average secondary particle diameter of 153.2 $\mu$m, and an angle of repose of 30°.

Comparative Example 1

Magnesium hydroxide particles, "Kisuma", manufactured by Kyowa Chemical Industry Co., Ltd. were used as a binder. The BET specific surface area was 13.5 $m^2/g$, and the angle of repose was 51°.

Comparative Example 2

Magnesium carbonate particles, "Shita", manufactured by Kyowa Chemical Industry Co., Ltd. were used as a binder. The BET specific surface area was 51.3 $m^2/g$, and the angle of repose was 47°.

Comparative Example 3

Magnesium silicate particles, "Torifu", manufactured by Kyowa Chemical Industry Co., Ltd. were  used as a binder. The BET specific surface area was 283 $m^2/g$, and the angle of repose was 44°.

Comparative Example 4

Crystalline cellulose, "Avicel PH101", manufactured by Asahi Kasei Chemicals Corporation was used as a binder. The BET specific surface area was 1.9 m$^2$/g, and the angle of repose was 42°.

<Compression test>

Mixtures were prepared in accordance with the prescriptions shown in Table 1 for each binder in Examples 1 and 2 and Comparative Examples 1 to 4 and were compressed into tablets each having a diameter of 8 mm and a weight of 250 mg at a tableting pressure of 600 kgf with a rotary tableting machine (VIRG, manufactured by Kikusui Seisakusho Ltd.). The physical properties of the resulting tablets are shown in Table 2.

Table 1

| Prescription Name / Ingredient | Model prescription (proportion of 0%) | Proportion of 10% | Proportion of 15% | Proportion of 20% |
|---|---|---|---|---|
| Ethenzamide | 10.0% | 10.0% | 10.0% | 10.0% |
| Anhydrous calcium phosphate | 84.0% | 74.0% | 69.0% | 64.0% |
| Binder | 0% | 10.0% | 15.0% | 20.0% |
| Croscarmellose sodium | 5.0% | 5.0% | 5.0% | 5.0% |
| Magnesium stearate | 1.0% | 1.0% | 1.0% | 1.0% |
| Total | 100.0% | 100.0% | 100.0% | 100.0% |

Ethenzamide: "Ethenzamide powder product" manufactured by Iwaki & Co., Ltd.

Anhydrous calcium phosphate: "Anhydrous calcium phosphate GS" manufactured by Kyowa Chemical Industry Co., Ltd.

Croscarmellose sodium: "Kiccolate ND-2HS" manufactured by Nichirin Chemical Industries, Ltd.

Magnesium stearate: "Plant magnesium stearate" manufactured by Taihei Chemical Industrial Co., Ltd.

Table 2

| Binder | Prescription Name | Tablet hardness (N) | Disintegration time (sec) | Friability (%) |
|---|---|---|---|---|
| Example 1 | 10% | 35 | 6 | 0.34 |
| | 15% | 53 | 7 | 0.08 |
| Example 2 | 10% | 27 | 8 | 0.60 |
| | 15% | 46 | 8 | 0.29 |
| | 20% | 57 | 8 | 0.26 |
| Comparative Example 1 | 20% | 15 | 6 | 1.51 |
| Comparative Example 2 | 20% | 28 | 7 | 0.82 |
| Comparative Example 3 | 20% | 18 | 9 | 1.08 |
| Comparative Example 4 | 15% | 41 | 8 | 0.52 |
| | 20% | 51 | 9 | 0.34 |
| Model prescription | 0% | 13 | 7 | 3.39 |

As shown in Table 2, in Comparative Examples 1 to 3 using conventional magnesium compounds, though the content of each binder was 20%, the tablet hardness was less than 30 N, and the friability was 0.8% or more. The novel binders each consisting of carbonate-containing magnesium hydroxide particles of Example 1 or 2 showed high bondability equivalent to or higher than that of the crystalline cellulose of Comparative Example 4, which is an excellent binder that is widely used.

Advantageous Effects of Invention

The present invention provides a binder useful in production of, for example, drugs and foodstuffs. The binder composed of the carbonate-containing magnesium hydroxide particles of the present invention has a small primary particle size and a high BET specific surface area and is therefore a novel binder having excellent bondability and moldability. The spherically granulated binder of the present invention prepared by spray drying has considerably high fluidity and can thereby be formulated by direct compression. Even if the binder of the present invention is prescribed together with a disintegrant or a water-soluble excipient, the binder can improve the tablet hardness without elongating the disintegration time of the tablet. Furthermore, the binder of the present invention can be expected to stabilize agents unstable to acids.

**Claims**

1. A binder having a BET specific surface area of 80 to 400 $m^2/g$ and comprising carbonate-containing magnesium hydroxide particles represented by Formula (1):

$$Mg(OH)_{2-x}(CO_3)_{0.5x} \cdot mH_2O \qquad (1)$$

wherein, x and m satisfy the following requirements:

$$0.02 \leq x \leq 0.7,$$

and

$$0 \leq m \leq 1.$$

2. The binder according to Claim 1, wherein the binder has an average secondary particle diameter of 1 to 1000 $\mu m$.

3. A granulated binder prepared by granulating the binder according to Claim 1.

4. The granulated binder according to Claim 3, wherein granulation is performed by spray drying of a slurry, dry granulation, or wet granulation.

5. The granulated binder according to Claim 3, having an average secondary particle diameter of 20 to 1000 $\mu$m.

6. A compression molded product comprising at least one binder according to any one of Claims 1 to 5.

7. The compression molded product according to Claim 6, further comprising a disintegrant.

8. The compression molded product according to Claim 7, wherein the disintegrant is at least one selected from the group consisting of starch, croscarmellose sodium, crospovidone, carmellose calcium, carmellose, low-substituted hydroxypropyl cellulose, and carboxymethyl starch sodium.

9. The compression molded product according to Claim 6, further comprising a water-soluble excipient.

10. The disintegrating compression molded product according to Claim 9, wherein the water-soluble excipient is sugar, starch, sugar alcohol, or a water-soluble salt.

11. The compression molded product according to Claim 6, the compression molded product being formed by direct compression and having a tablet strength of 20 N or more.

12. The compression molded product according to Claim 6, the compression molded product being formed by direct compression and having a tablet strength of 30 N or more.

13. The compression molded product according to Claim 6, the compression molded product being formed by direct compression and having a friability of 0.5% or less.

14. The compression molded product according to Claim 6, comprising a drug efficacy ingredient.

15. The compression molded product according to Claim 14, wherein the drug efficacy ingredient is unstable to an acid.

16. A method of using carbonate-containing magnesium hydroxide particles having a BET specific surface area of 80 to 400 m$^2$/g and represented by the following Formula (1) as a binder for a compression molded product:

$$Mg(OH)_{2-x}(CO_3)_{0.5x} \cdot mH_2O \qquad (1)$$

wherein, x and m satisfy the following requirements:

$$0.02 \leq x \leq 0.7,$$

and

$$0 \leq m \leq 1.$$

## INTERNATIONAL SEARCH REPORT

| | International application No. |
|---|---|
| | PCT/JP2012/057414 |

### A. CLASSIFICATION OF SUBJECT MATTER

*A61K47/02*(2006.01)i, *A61K9/20*(2006.01)i, *A61K47/26*(2006.01)i, *A61K47/32*(2006.01)i, *A61K47/36*(2006.01)i, *A61K47/38*(2006.01)i

According to International Patent Classification (IPC) or to both national classification and IPC

### B. FIELDS SEARCHED

Minimum documentation searched (classification system followed by classification symbols)
A61K9/00-9/72, A61K47/00-47/48

Documentation searched other than minimum documentation to the extent that such documents are included in the fields searched
Jitsuyo Shinan Koho          1922-1996   Jitsuyo Shinan Toroku Koho   1996-2012
Kokai Jitsuyo Shinan Koho    1971-2012   Toroku Jitsuyo Shinan Koho   1994-2012

Electronic data base consulted during the international search (name of data base and, where practicable, search terms used)
CA/MEDLINE(STN)

### C. DOCUMENTS CONSIDERED TO BE RELEVANT

| Category* | Citation of document, with indication, where appropriate, of the relevant passages | Relevant to claim No. |
|---|---|---|
| A | WO 2008/123566 A1  (KYOWA CHEM IND CO., LTD.), 16 October 2008 (16.10.2008), claims 1 to 11; page 14, line 10 to page 15, line 2 & TW 200904757 A        & EP 2135845 A1 & KR 2010014349 A       & CN 101652323 A & US 2010/098781 A1     & JP 2009-509287 A & VN 22143 A | 1-16 |
| A | WO 2005/123040 A1  (SHIONOGI & CO., LTD.), 29 December 2005 (29.12.2005), claims 1 to 5, 8; paragraph [0011] & JP 2006-514826 A | 1-16 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 June, 2012 (12.06.12) | 19 June, 2012 (19.06.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

| **INTERNATIONAL SEARCH REPORT** | International application No. |
| --- | --- |
| | PCT/JP2012/057414 |

Claims 1-5 relate to a "binder". With respect to a "binder", in the field of pharmaceutical preparations alone, various types of binders are generally used in a granulation process, a tabletting process and so on and the binders are included within the scope of the "binder".

Some of the compression-shaped products according to the inventions described in claim 6-10 and 14-15, which refer to claims 1-5, are produced using a binder in a granulation process, a tabletting process and so on.

However, only examples in which the binder is used in a compression-shaped product produced by a direct tabletting technique is disclosed in the meaning within PCT Article 5 in the present application. Further, in claims 1-5, 6-10 and 14-15, any specific embodiment for using the binder according to the inventions described in claims 1-5 or any preparation containing the binder is not described clearly. Therefore, it cannot be considered that claims 1-5, 6-10 and 14-15 are supported by the disclosure of the description in the meaning within PCT Article 5.

Such being the case, the search was carried out on the binder which can be used in a compression-shaped product produced by a direct tabletting technique.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI7118005 B
- WO 2008123566 A
- JP 3746167 B
- JP 2009209048 A
- JP 2010047553 A
- JP 2008255088 A
- JP 2008174500 A